# EUROPEAN PATENT SPECIFICATION

(11) **EP 1 757 943 B1**
(45) Date of publication and mention of the grant of the patent: **20.10.2010**
(21) Application number: 06090144.4
(22) Date of filing: 18.08.2006
(51) Int. Cl.: G01N 33/68, G01N 33/543, C07K 17/00

(54) **Array for identification of protein-protein interactions**
Anordnung zur Identifizierung von Protein-Protein-Interaktionen
Réseau pour l'identification d'interactions protéine-protéine

(30) Priority: 26.08.2005 EP 05090248
(43) Date of publication of application: 28.02.2007
(73) Proprietor: MAX-DELBRÜCK-CENTRUM FÜR MOLEKULARE MEDIZIN, 13125 Berlin (DE)
(72) Inventor: Wanker, Erich, 13187 Berlin (DE); Grelle, Gerlinde, 13125 Berlin (DE); Kostka, Susanne, 13125 Berlin (DE); Otto, Albrecht, 13125 Berlin (DE); Genser, Klaus, 9020 Klagenfurt (AT); Strödicke, Martin, 12163 Berlin (DE)
(74) Representative: Boeckh, Tobias

(56) References cited:
- US-A1- 2004 161 748
- SNAPYAN MARINA ET AL: "Dissecting DNA-protein and protein-protein interactions involved in bacterial transcriptional regulation by a sensitive protein array method combining a near-infrared fluorescence detection." PROTEOMICS. MAY 2003, vol. 3, no. 5, May 2003 (2003-05), pages 647-657, XP002355537 ISSN: 1615-9853
- BÜSSOW K ET AL: "A human cDNA library for high-throughput protein expression screening." GENOMICS. 1 APR 2000, vol. 65, no. 1, 1 April 2000 (2000-04-01), pages 1-8, XP002355538 ISSN: 0888-7543
- GE H: "UPA, a universal protein array system for quantitative detection of protein-protein, protein-DNA, protein-RNA and protein-ligand interactions." NUCLEIC ACIDS RESEARCH. 15 JAN 2000, vol. 28, no. 2, 15 January 2000 (2000-01-15), page e3, XP002355539 ISSN: 1362-4962
- LUEKING ANGELIKA ET AL: "Protein microarrays: A tool for the post-genomic era" CURRENT GENOMICS, vol. 2, no. 2, June 2001 (2001-06), pages 151-159, XP008055889 ISSN: 1389-2029
- LUEKING A ET AL: "Protein microarrays for gene expression and antibody screening" ANALYTICAL BIOCHEMISTRY, ACADEMIC PRESS INC, NEW YORK, vol. 270, 1 January 1999 (1999-01-01), pages 103-111, XP002127382 ISSN: 0003-2697

## Description

The present invention relates to a proteomics approach for systematic identification of protein-protein interactions (e.g. in a unpurified probe of body fluid); the invention is also directed to a method for screening of protein-protein interactions. Additionally, the present disclosure provides an array - e.g. a chip, a bead or a micro-plate - and furthermore the use of the array for detection of said interaction.

Many proteins mediate their biological function through interactions with other proteins (1). Therefore, the systematic identification and characterization of protein-protein interactions (PPIs) has become a powerful proteomics strategy to understand protein function and comprehensive cellular regulatory networks (2-4).

Because protein arrays allow the parallel detection of addressable elements in a single experiment (5-11), they have been recognized as a promising technology for the identification of PPIs (12,13) or other specific biochemical activities (13-16), and much effort has been devoted to their development in recent years. Zhu et al. (13), e.g., printed 5,800 purified yeast proteins onto coated glass slides and employed them successfully in proof-of-principle experiments for the detection of novel calmodulin- and phospholipid-interacting proteins.

Protein arrays were also used efficiently for identifying novel targets of phosphodiesterases, methyltransferases (17) and protein kinases (18,19). In principle, the production of protein arrays and their utilization for systematic large-scale interaction and activity screens has proven viable. One major drawback, however, has been the necessity to use purified proteins. Protein purification is usually difficult, time consuming and expensive. For array-based studies, especially high-throughput systematic screening (12), technologies are needed that permit the use of crude protein extracts.

Lueking et al (47) describe a method to produce a protein microarray as well as the use of said array for molecule interaction analyses. However the analyses of molecule interactions based on the use of crude (ie non-purified) cell lysates, is not described by these authors.

At present, there are no arrays available for detection of interaction of raw or unpurified proteins in a probe; preferred in a biological or chemical probe. Therefore, the technical problem underlying the present invention is to provide methods which allow the screening of protein-protein interaction in crude cell extracts.

This problem is solved by the provision of the embodiments as defined in the claims.

It has been surprisingly discovered that methods of screening of specific protein-protein interactions comprising the following steps:
(a) providing a library of cDNA clones using a first acceptable carrier, allowing the expression of a first protein,
(b) expression of the first protein,
(c) providing a library of cDNA clones using a second acceptable carrier, allowing the expression of a second protein or
(d) expression of the second protein of (c),
(e) lysis and centrifugation/ filtration of the clones used in step (a), whereby a crude cell extract comprising the first protein is obtained,
(f) lysis and centrifugation/ filtration of the clones used in step (c) or (c'), whereby a crude cell extract comprising the second protein is obtained,
(g) immobilisation of the crude cell extract comprising the expressed first protein obtained in step (e) using a third acceptable carrier,
(h) contacting the third carrier with the crude cell extract obtained in step (f) or with the probe in step (c') comprising the second protein,
(i) detection of the interaction of the first and the second protein, whereat the interaction of proteins in step (i) is detected using an antibody against a protein-tag of the second protein obtained in step (f) and / or using an antibody against the second protein.
are suitable for probes of crude protein extracts or unpurified recombinant human proteins.

The present invention provides a novel method for the parallel screening of mammalian protein-protein interactions using high-density spotted protein arrays. The present disclosure will also offer arrays which are suitable for detection of interactions of proteins in crude cell extracts.

The method and/or the array have several advantages: Unpurified recombinant human proteins, produced in bacteria under nondenaturating conditions, can be used as baits as well as preys for interaction screens. The screening procedure is simple, sensitive and does not require radioactivity or expensive equipment, and is therefore particularly useful for systematic high-throughput interaction studies of human proteins.

Proteins mediate their biological function through interactions with other proteins. Therefore, the systematic identification and characterization of protein-protein interactions (PPIs) have become a powerful proteomic strategy to understand protein function and comprehensive cellular regulatory networks. The proteome array technology of the invention permits the high-throughput identification of such PPIs that are functionally involved in neurodegenerative, cancer, cardiovascular, as well as other human disorders. The high-throughput identification of protein-protein and protein-DNA interactions allows the generation of interaction networks, which facilitate the assignment of so far uncharacterized proteins to cellular pathways. Subsequently, the high-throughput identification of protein-drug interactions lead to the identification of compouds interfering with such regulatory protein networks to give a first hint for the development of new disease therapies.

Here we described a protein array-based technology for high-throughput screening of potential PPIs. The method has several advantages. Unpurified recombinant proteins, produced in bacteria under non-denaturing conditions in 384-well plates, can be used for interaction screens. The screening procedure is simple and sensitive, does not require radioactivity or expensive equipment, and is therefore particularly useful for systematic high throughput interaction studies of human proteins as well as proteins of other organisms. In contrast to the method of the invention, the state of the art and especially Synapyan et al. published a different strategy, which is completely unsuitable to perform a high-throughput protein-protein interaction screen. Following points are different to our method and argue against the high-throughput application.
1. The methods of the state of the art, especially of Synapyan et al is not feasible with a crude cell extract. Therefore, according to Synapyan, it is essential to isolate the protein. This is time consuming and expensive in a high througput approache. In contrast, the array or the method of the invention allows the use of crude cell extracts containing the overexpressed overlay protein.
2. In the methods of the state of the art, especially of Synapyan et al., the purified overlay protein must be directly labled, which is also time consuming and expensive in a high througput approache. According to prior art, especially to Synapyan et al. it is not possible to lable a crude cell extract. The method according to Synapyan requires the labelling of purified proteins. In the method of the invention, the overlay protein is identified using an antibody against the protein-tag.
3. In contrast to Synapyan et al. in the context of the invention microtiter plates (MTPs) are used as carrier for clone libraries as a prerequisites for a systematic high-throughput interaction screen. In such microtiter plates each well contains an individual clone. In the method of Synapyan et al. on the other hand no isolated material is used.
4. In contrast to prior art and especially to Synapyan et al. it is possible to miniaturize the protein arrays (micro arrays) to increase the efficiency the screening procedure.

Together this indicates that the efficiency of our method in a high throughput approach is dramatically increased compaired to the method of Synapyan et al.. The method according to Synapyan et al. cannot be used or applied as a high throughput approach.

Ge, H., Nucleic Acids Research, 2000, also describes a method for the production of arrays. However, this array can only be used with purified proteins. A high-throughput application is therefore impossible with this method.

US 2004/161748 discloses a protein array. However, crude cell extracts cannot be analysed with this array.

The teachings of the disclosure are further characterized by the following features:
- departure from the beaten track
- a new perception of the problem
- satisfaction of a long-felt need or want
- hitherto all efforts of experts were in vain
- the simplicity of a solution proves inventive action, especially if it replaces a more complex doctrine
- the development of scientific technology followed another direction
- the achievement forwards the development
- misconceptions about the solution of the according problem (prejudice)
- technical progress, such as: improvement, increased performance, price-reduction, saving of time, material, work steps, costs or resources that are difficult to obtain, improved reliability, remedy of defects, improved quality, no maintenance, increased efficiency, better yield, augmentation of technical possibilities, provision of another product, opening of a second way, opening of a new field, first solution for a task, spare product, alternatives, possibility of rationalisation, automation or miniaturisation or enrichment of the pharmaceutical fund
- special choice (since a certain possibility, the result of which was unforeseeable, was chosen among a great number of possibilities, it is a patentable lucky choice)
- error in a citation
- young field of technology
- combined invention; a combination of a number of known elements, with a surprising effect
- licensing
- praise of experts and
- commercial success

It is preferred that the clones in step (a) are E.coli clones, yeast clones or clones using mammalian cells. Those skilled in the art are familiar with these clones and using thereof.

In yet other embodiments, the invention relates to a method, whereby the first carrier in step (a) is a microtiter plate and/or the second carrier of step (c) is a membrane filter or a coated glass slide.

In a further preferred embodiment the invention relates to a method, whereby the lysis in step (e) and/or (f) is a native cell lysis, in particular using lysozyme or ultrasonic. Those skilled in the art will be familiar with numerous such lysis methods.

It is also preferred that the crude cell extract comprising the first protein of step (e) is spotted onto a membrane or a coated glass slide.

In another preferred embodiment the cell extracts in steps (e) and/or (f) are native cell extracts. In a more preferred method the contacting step (h) is an overlay assay.

It is preferred that the interaction of proteins in step (i) is detected using an antibody against a protein-tag of the second protein obtained in step (f) and / or using an antibody against the second protein. It is further preferred that, the protein-tag of the second protein obtained in step (f) is a GST-, HIS-, myc-, FLAG-, HA- or a fluorescenttag.

In a preferred embodiment of the invention the interaction of the proteins in step (i) is detected by a fluorescence or an enzyme-linked detection or method.

The disclosure relates also to a method for manufacturing of an array comprising the following steps:
(a) providing a library of cDNA clones using a first acceptable carrier, allowing the expression of a first protein,
(b) expression of the first protein,
(c) lysis and centrifugation/filtration of the clones, whereby a crude cell extract comprising the first protein is obtained and
(d) immobilisation of the crude cell extract comprising the first protein using a second acceptable carrier.

Also disclosed is (i) an array manufactured by the method for manufacturing of an array and an (ii) array for in-vitro detection of a interaction of a first protein with a second protein, whereby an arrayed crude cell extract contains the first protein. The present disclosure also relates to use of the array for detection of protein-protein interaction. An "array" is an arrangement of entities in a pattern on a substrate. Although the pattern is typically a two-dimensional pattern, the pattern may also be a three-dimensional pattern. Typically, the protein arrays comprise micrometer-scale, two-dimensional patterns of patches of proteins immobilized on an organic thinfilm coating on the surface of the substrate. In one example, the present disclosure provides an array of proteins (and/or crude cell extract) which comprises a substrate, at least one organic thinfilm on some or all of the substrate surface, and a plurality of patches arranged in discrete, known regions on portions of the substrate surface covered by organic thinfilm, wherein each of said patches comprises a protein immobilized on the underlying organic thinfilm.

In most cases, the array will comprise at least about ten patches. In a preferred example, the array comprises at least about 50 patches. In a particularly preferred example the array comprises at least about 100 patches. In alternative preferred examples, the array of crude cell extract may comprise more than 10.sup.3, 10.sup.4 or 10.sup.5 patches. The area of surface of the substrate covered by each of the patches is preferably no more than about 0.25 mm.sup.2. Preferably, the area of the substrate surface covered by each of the patches is between about 1 .mu.m.sup.2 and about 10,000 .mu.m.sup.2. In a particularly preferred example, each patch covers an area of the substrate surface from about 100 .mu.m.sup.2 to about 2,500 .mu.m.sup.2. In an alternative example, a patch on the array may cover an area of the substrate surface as small as about 2,500 nm.sup.2, although patches of such small size are generally not necessary for the use of the array.

The patches of the array may be of any geometric shape. For instance, the patches may be rectangular or circular. The patches of the array may also be irregularly shaped.

The distance separating the patches of the array can vary. Preferably, the patches of the array are separated from neighboring patches by about 1 .mu.m to about 500 .mu.m. Typically, the distance separating the patches is roughly proportional to the diameter or side length of the patches on the array if the patches have dimensions greater than about 10 .mu.m. If the patch size is smaller, then the distance separating the patches will typically be larger than the dimensions of the patch.

In a preferred example of the array, the patches of the array are all contained within an area of about 1 cm.sup.2 or less on the surface of the substrate. In one preferred example of the array, therefore, the array comprises 100 or more patches within a total area of about 1 cm.sup.2 or less on the surface of the substrate. Alternatively, a particularly preferred array comprises 10.sup.3 or more patches within a total area of about 1 cm.sup.2 or less. A preferred array may even optionally comprise 10.sup.4 or 10.sup.5 or more patches within an area of about 1 cm.sup.2 or less on the surface of the substrate. In other examples, all of the patches of the array are contained within an area of about 1 mm.sup.2 or less on the surface of the substrate.

Typically, only one type of crude cell extract/protein is immobilized on each patch of the array. In a preferred example of the array, the crude cell extract/protein immobilized on one patch differs from the crude cell extract/protein immobilized on a second patch of the same array. In such an example, a plurality of different crude cell extract/proteins are present on separate patches of the array. Typically the array comprises at least about ten different proteins or crude cell extracts. Preferably, the array comprises at least about 50 different proteins. More preferably, the array comprises at least about 100 different proteins. Alternative preferred arrays comprise more than about 10.sup.3 different proteins or more than about 10.sup.4 different proteins. The array may even optionally comprise more than about 10.sup.5 different proteins (in the context of the invention the term protein is also used as synonym for the term crude cell extract).

In one example of the array, each of the patches of the array comprises a different protein. For instance, an array comprising about 100 patches could comprise about 100 different proteins. Likewise, an array of about 10,000 patches could comprise about 10,000 different proteins. In an alternative example, however, each different protein is immobilized on more than one separate patch on the array. For instance, each different protein may optionally be present on two to six different patches. An array, therefore, may comprise about three-thousand protein patches, but only comprise about one thousand different proteins since each different protein is present on three different patches.

In another example, although the protein of one patch is different from that of another, the proteins are related. In a preferred example, the two different proteins are members of the same protein family. The different proteins on array may be either functionally related or just suspected of being functionally related. In another array, however, the function of the immobilized proteins may be unknown. In this case, the different proteins on the different patches of the array share a similarity in structure or sequence or are simply suspected of sharing a similarity in structure or sequence. Alternatively, the immobilized proteins may be just fragments of different members of a protein family.

The proteins immobilized on the array may be members of a protein family such as a receptor family (examples: growth factor receptors, catecholamine receptors, amino acid derivative receptors, cytokine receptors, lectins), ligand family (examples: cytokines, serpins), enzyme family (examples: proteases, kinases, phosphatases, ras-like GTPases, hydrolases), and transcription factors (examples: steroid hormone receptors, heat-shock transcription factors, zinc-finger proteins, leucine-zipper proteins, homeodomain proteins). In one example, the different immobilized proteins are all HIV proteases or hepatitis C virus (HCV) proteases. In other examples, the immobilized proteins on the patches of the array are all hormone receptors, neurotransmitter receptors, extracellular matrix receptors, antibodies, DNA-binding proteins, intracellular signal transduction modulators and effectors, apoptosis-related factors, DNA synthesis factors, DNA repair factors, DNA recombination factors, or cell-surface antigens.

The term "bead" means a small particle, generally less than about 1 mm (e.g., less than about 100 .mu.m) in any dimension, with the ability to have reagents attached to its surface or stored in its interior. A bead can be made from one or more of a variety of materials, including organic polymers, glass, and metals. The reagent is typically attached to the bead by chemical reaction with a reactive functional group such as a carboxyl, silanol, or amino group on its surface. Reagents can, for example, be confined to the bead by covalent chemical attachment or by physical adsorption to the bead surface. The bead shape can be nearly spherical, irregularly shaped, or of an intermediate shape. The term "stringency" refers to the degree to which non-specific molecular interactions are disrupted during a washing step. "Specific interactions" are interactions between two molecules resulting from a unique three-dimensional structure of at least one of the molecules involved. For example, enzymes have specific interactions with transition state analogues due to their evolution toward stabilizing reaction intermediates. The term "micro-plate" refers to a collection plate used to transfer the contents of the through-holes of an array, where no cross contamination of the through-holes occurs in the transfer.

Unless otherwise defined, all technical and scientific terms used herein have the same meaning as commonly understood by a person of ordinary skill in the art to which this invention belongs. Although methods and materials similar or equivalent to those described herein can be used in the practice or testing of the present disclosure, suitable methods and materials are described below. .

The term "probe" or "biological/chemical probe" refers to a chemical, biological, mechanical, or electronic structure that detects a specific analyte by a specific binding or catalytic event.

The binding or catalytic event can be transduced into a signal readable by an operator. One type of chemical probe is an affinity probe (e.g., a specific protein that binds to another protein). Examples of mechanical probes include a cantilever that has a ligand immobilized on its surface and a material whose properties (e.g., strain, inertia, surface tension) change in response to a chemical or biological event.

The term "chemical detection event" refers to a chemical reaction between molecule(s) of interest and probe molecule(s) that in turn produces a signal that can be observed by an operator. For example, the hydrolysis of fluorescein di-.beta.-galactoside by the enzyme .beta.-galactosidase, to produce the fluorescent molecule fluorescein, is a chemical detection event. In some cases, the chemical detection event can involve a series of chemical reactions triggered by an initial interaction of analyte and probe (e.g., activation of a signal transduction pathway in a probe cell by the binding of a ligand to a surface receptor).

The term "linker molecule" means a molecule that has a high affinity for or covalently links to the surface of a platen or bead. The linker molecule can have a spacer segment such as a carbon chain, and can also have a functional group at its end to enable attachment of probe molecules covalently or with high affinity.

"Proteomics" means the study of or the characterization of either the proteome or some fraction of the proteome. The "proteome" is the total collection of the intracellular proteins of a cell or population of cells and the proteins secreted by the cell or population of cells. This characterization most typically includes measurements of the presence, and usually quantity, of the proteins which have been expressed by a cell. The function, structural characteristics (such as post translational modification), and location within the cell of the proteins may also be studied. "Functional proteomics" refers to the study of the functional characteristics, activity level, and structural characteristics of the protein expression products of a cell or population of cells.

A "protein" means a polymer of amino acid residues linked together by peptide bonds. The term, as used herein, refers to proteins, polypeptides, and peptides of any size, structure, or function. Typically, however, a protein will be at least six amino acids long. Preferably, if the protein is a short peptide, it will be at least about 10 amino acid residues long. A protein may be naturally occurring, recombinant, or synthetic, or any combination of these. The term protein may also apply to amino acid polymers in which one or more amino acid residues is an artificial chemical analogue of a corresponding naturally occurring amino acid. An amino acid polymer in which one or more amino acid residues is an "unnatural" amino acid, not corresponding to any naturally occurring amino acid, is also encompassed by the use of the term "protein" herein.

A "body fluid" may be any liquid substance extracted, excreted, or secreted from an organism or tissue of an organism. The body fluid need not necessarily contain cells. Body fluids of relevance to the present invention include, but are not limited to, whole blood, serum, urine, plasma, cerebral spinal fluid, tears, sinovial fluid, and amniotic fluid.

A "fragment of a protein" means a protein which is a portion of another protein. For instance, fragments of a proteins may be polypeptides obtained by digesting full-length protein isolated from cultured cells. A fragment of a protein will typically comprise at least six amino acids. More typically, the fragment will comprise at least ten amino acids. Preferably, the fragment comprises at least about 16 amino acids.

The term "immobilized" means substantially attached at the molecular level (i.e., through a covalent or non-covalent bond or interaction). Variables in immobilisation of proteins/crude cell extracts include both the coupling reagent and the nature of the surface being coupled to. The properties of a good protein array support surface are that it should be chemically stable before and after the coupling procedures, allow good spot morphology, display minimal nonspecific binding, not contribute a background in detection systems, and be compatible with different detection systems. The immobilisation method used should be reproducible, applicable to proteins of different properties (size, hydrophilic, hydrophobic), amenable to high throughput and automation, and compatible with retention of fully functional protein activity. Orientation of the surface-bound protein is recognised as an important factor in presenting it to ligand or substrate in an active state; for capture arrays the most efficient binding results are obtained with orientated capture reagents, which generally requires site-specific labelling of the protein.

Passive adsorption to surfaces is methodologically simple, but allows little quantitative or orientational control; it may or may not alter the functional properties of the protein, and reproducibility and efficiency are variable. Covalent coupling methods provide a stable linkage, can be applied to a range of proteins and have good reproducibility; however, orientation may be variable, chemical derivatisation may alter the function of the protein and requires a stable interactive surface. Biological capture methods utilising a tag on the protein provide a stable linkage and bind the protein specifically and in reproducible orientation, but the biological reagent must first be immobilised adequately and the array may require special handling and have variable stability. Several immobilisation chemistries and tags have been described for fabrication of protein arrays. Substrates for covalent attachment include glass slides coated with amino- or aldehyde-containing silane reagents. In the Versalinx system, reversible covalent coupling is achieved by interaction between the protein derivatised with phenyldiboronic acid, and salicylhydroxamic acid immobilised on the support surface. This also has low background binding and low intrinsic fluorescence and allows the immobilised proteins to retain function. Noncovalent binding of unmodified protein occurs within porous structures such as HydroGel, based on a 3-dimensional polyacrylamide gel; this substrate is reported to give a particularly low background on glass microarrays, with a high capacity and retention of protein function. Widely used biological coupling methods are through biotin/streptavidin or hexahistidine/Ni interactions, having modified the protein appropriately. Biotin may be conjugated to a poly-lysine backbone immobilised on a surface such as titanium dioxide or tantalum pentoxide.

The term "binding partner" means a protein which is bound by a particular protein-capture agent, preferably in a substantially specific manner. In some cases, the binding partner may be the protein normally bound in vivo by a protein which is a protein-capture agent. In other embodiments, however, the binding partner may be the protein or peptide on which the protein-capture agent was selected (through in vitro or in vivo selection) or raised (as in the case of antibodies). A binding partner may be shared by more than one protein-capture agent. For instance, a binding partner which is bound by a variety of polyclonal antibodies may bear a number of different epitopes. One protein-capture agent may also bind to a multitude of binding partners (for instance, if the binding partners share the same epitope).

"Conditions suitable for protein binding" means those conditions (in terms of salt concentration, pH, detergent, protein concentration, temperature, etc.) which allow for binding to occur between a protein and its binding partner in solution. Preferably, the conditions are not so lenient that a significant amount of nonspecific protein binding occurs.

The invention will be explained in more detail with reference to the following examples.

### EXPERIMENTS

We have applied our technology in poof-of-principle experiments to identify novel interaction partners for the proteins CHIP, amphiphysin II and VCP. CHIP is a co-chaperone that associates with heat shock proteins such as Hsp70 and Hsp90 (40,41). Moreover, it functions as an E3 ligase in order to polyubiquitinate target proteins (25,29). The overlay screen revealed that CHIP interacts with Hsp70, Hsc70 and Hsp90 as well as with the microtubule associated protein Tau, confirming previous studies (29,40). Moreover, novel interactions with the proteins caytaxin and E2Q were identified (Table 1). Recent studies have demonstrated that mutants in caytaxin cause Cayman disease, an inheritable recessive ataxia restricted to the area of Grand Cayman Island (30). Currently, the molecular mechanism causing Cayman ataxia is completely unclear. Our findings that caytaxin associates with CHIP suggests that it might be a substrate for CHIP-mediated ubiquitination. To test this hypothesis in vitro, ubiquitination assays were performed indeed showing that CHIP stimulates the polyubiquitination of caytaxin. Interestingly, the in vitro ubiquitination was not dependent on the presence of Hsc70. This finding substantiates that the identified CHIP-caytaxin interaction is direct and not mediated by a chaperone. These results suggest that CHIP may influence the ubiquitination rate and turnover of caytaxin in neurons of healthy individuals as well as Cayman disease patients.

The novel CHIP interacting protein E2Q is a potential ubiquitin conjugating enzyme (E4) not yet characterized. To date, the functional relevance of the CHIP/E2Q interaction is unknown. However, it seems reasonable to speculate that CHIP may use E2Q for efficient ubiquitination of certain target proteins.

Using the SH3 domain (aa 497-593) as a bait, we identified three novel partner proteins for the synaptic protein amphiphysin II by protein array overlay screening. These interactions could be validated by pull-down and Y2H assays. For example, an association between the SH3 domain with the Discs large-associated protein DLP4 was established in this study. DLP4 has been shown to function in membrane morphogenesis and endocytosis in the postsynaptic density of neurons. As amphiphysin II colocalizes with the marker Discs large (Dlg) in Drosophila (42,43) and functions in vesicle transport processes in postsynaptic densities, we suggest that a functional DLP4/amphiphysin II protein complex influencing synaptic activities could form in neurons.

Two other novel interactions of the SH3 domain of amphiphysin II, namely with the DNA double-strand break repair protein XRCC4 and the fructose-1,6-bisphosphatase FBP were detected by array screening. XRRC4 is localized in the nucleus and critical for non-homologous end joining of broken chromosomal DNA fragments, while FBP is crucial for glyconeogenesis, the production of glucose from non-carbohydrate precursors such as pyruvate in the cytosol (43). Whether amphiphysin II, via interactions, can modulate XRCC4 or FBP activity needs to be analysed.

It is generally thought that SH3 domains associate with relative short proline-rich peptide motifs in their target proteins (44). Therefore, we mapped the binding regions in DLP4, XRCC4 and FBP using peptide arrays (26). Interestingly, we found that the SH3 domain of amphiphysin II not only interacts with proline-rich sequences of the known core PxxP element (34), but also with R/K-rich sequences, which were detected in XRCC4. This suggests that basic amino acids can be recognized by SH3 domains and are critical for the formation of PPIs in vitro.

Finally, we searched for interaction partners of the hexameric VCP protein, which mainly functions as an unfoldase in membrane fusion events (45) and the transport and degradation of ubiquitinated proteins (39,46). Our approach allowed the identification of two known VCP interactors, p47 (37) and AMFR (39), which were both validated using a whole set of in vitro and in vivo binding experiments. Using pull-down assays, the binding regions for the proteins VCP and AMFR were mapped. Interestingly, the C-terminus of AMFR is necessary and sufficient for the interaction with the N-terminus of VCP. Further studies are needed to address the question whether AMFR targets VCP to ER membranes in vivo and modulates the degradation of polyubiquitinated substrates.

Jointly, these results indicate that our array screening approach permits the identification of partners for different types of bait proteins which are expressed in soluble form in E. coli. The screening procedure has the following advantages: First, recombinant proteins can be expressed in small-scale in a 384-well format and have no longer to be purified for in vitro interaction studies. This dramatically reduces the costs and enhances the speed of protein production. Second, using our current robotic system, up to 27,648 individual protein extracts can be double spotted in a 5x5 gridding pattern onto a filter and identical arrays can be generated simultaneously. This enables high-throughput production of protein arrays for overlay screens. Third, the spotting procedure is highly reproducible and the use of double spots reduces the number of false positives. In this study, the interaction partners of CHIP, amphiphysin II and VCP were identified in three different overlay experiments, underlining the reliability of the method. Fourth, overlay identification of interactions is a simple membrane-based ELISA assay and does not require radioactivity. Fifth, bacterial cell extracts containing GST-tagged human fusion proteins can be used for the overlay procedure. However, also untagged recombinant proteins as well as TAP-, FLAG-, or MBP-tagged fusions may be utilized for screening. Sixth, our method is generic and not limited to identifying human PPIs. We propose that also expression clones encoding proteins from other higher organisms such as mouse, rat or Drosophila can be screened. Moreover, the technology is likely to be applicable for protein-DNA, protein-drug as well as protein profiling studies.

**Clones and constructs**. For the production of protein arrays, clones from the hEx1 library (20) expressing His-tagged human fusion proteins were used. For the expression of GST (glutathione S-transferase)-tagged fusions, cDNA fragments encoding CHIP (carboxy terminus of the HSC70 interacting protein) (aa 2-303) and amphiphysin II (aa 497-593) were PCR-amplified from the brain cDNA pool No. 588 (RZPD, Berlin, Germany), and subcloned into the plasmids pGEX-6P-1 and -2 (Pharmacia), respectively. The cDNA encoding VCP (Valosin-containing protein) was obtained from ATCC (American Type Culture Collection), amplified and subcloned into pGEX-6P-1 (Pharmacia) and pTL-1 (21) for expression in E. coli and mammalian cells, respectively. Dynamin1-encoding cDNA (aa 634-864) was amplified from full-length cDNA obtained from RZPD (Deutsches Ressourcenzentrum für Genomforschung GmbH; Berlin, Germany) and subcloned into pQE30-NST. For co-localization studies, a cDNA fragment encoding AMFR (autocrine motility factor receptor) (aa 396-643) was subcloned into pTL-HA (hemagglutinin)-2 (21). For confirmation of interactions with the two-hybrid system, the plasmids pBTM117c (22) and pACT4 (derivative of Clontech's pACT2) were used.

**Sequence analysis**. Clones were subjected to DNA tag sequencing, starting from the 5'-ends of the cDNA inserts. After base calling and quality clipping of sequencer trace data (PHRED, trim_cutoff= 0.05), 13,964 sequences were translated into protein sequences. BLASTP searches in the nr (NCBI) and TrEMBL (Swissprot) protein databases were performed.

**Overexpression of His-tagged proteins for array production**. For expression of His-tagged human fusion proteins, 384-well microtiter plates were filled with 40 µl TB-medium per well (100 mM glucose, 100 µg/ml ampicillin and 15 µg/ml kanamycin), and inoculated with 13,824 E. coli clones from the hEx1 cDNA library. Cells were grown for 3 hours at 37°C, followed by induction of protein expression by addition of 40 µl 1 mM IPTG (isopropyl-thiogalactoside) and overnight incubation at 30°C. Then, cells were pelleted by centrifugation for 10 min at 4,000 rpm and 4°C and stored at -80°C.

**Native cell lysis and generation of protein arrays.** Cell pellets were resuspended in 50 µl lysis buffer (1 mg/ml lysozyme, 0.5% NP-40, 1% Triton X-100, 1 mM PMSF, 25 U/ml benzonase and 150 mM NaCl in PBS) and incubated for 90 min on ice. During incubation, cell suspensions were mixed carefully every 10 min using a 384-pin replicator. Then, the plates were centrifuged at 4,000 rpm and 4°C for 1 h to pellet cell debris. Supernatants were spotted onto 225 mm x 225 mm nitrocellulose membranes (Schleicher&Schuell/Protran BA 83) using the K2-003 Gridder (KBiosystems). Each extract was spotted 20 times on each position in a 5x5 duplicate gridding pattern.

**Overlay assay with GST-tagged fusion proteins.** GST fusion proteins were expressed in E. coli as described (23). Crude E. coli cell extracts were used for overlay screens. Membranes with high-density spotted His-tagged fusion proteins were blocked for 4 h at 4°C with PBS-T (PBS supplemented with 0.05% Tween 20) containing 0.5 mM DTT and 5% non-fat dry milk powder. Then, they were rinsed with PBS-T and incubated overnight at 4°C in 100 ml overlay buffer (PBS-T, supplemented with 5% fetal calf serum, 100 µM ATP and 100 µM GTP) to which crude bacterial cell extract containing the GST fusion protein (~0.5 mg) had been added prior to use. After incubation, membranes were washed once for 10 min with PBS-TT (TT = 0.05% Tween 20 + 0.2% Triton X-100) and three times for 10 min each with PBS-T. Subsequently, filters were incubated for 1 h at room temperature (RT) in 100 ml PBS-T containing anti-GST-HRP-conjugated antibody (5,000-fold diluted, Amersham Biosciences). Membranes were rinsed twice with PBS-T, washed once with PBS-T for 15 min and then three times for 5 min. Subsequently, filters were incubated for 3 min in CHEMIGLOW solution (Alphainnotech) according to the manufacturer's instructions. Signals were detected using a Fuji Luminescent Image Analyzer (LAS 1000 CH).

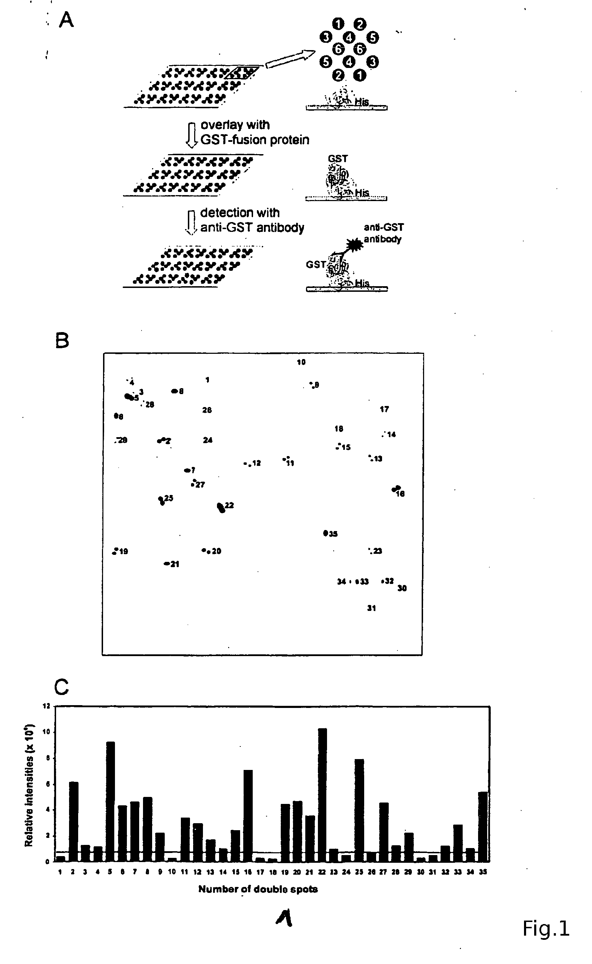

The resulting images were analysed with Aida Image Analyzer v. 3.21.001 (Raytest GmbH). The intensity of spots was determined using a spot diameter of 2,280 µm if both spots of a respective duplicate gave a distinct signal in the image. Background correction of the average signal intensities of duplicates was performed block-wise. Interactions were considered positive when the relative signal of respective duplicates was greater than three times the standard deviation of average background signal (Fig. 1C).

**In vitro protein-protein binding assays.** For pull-down experiments, ~20 µg GST fusion protein was immobilized on 100 µl glutathione-agarose beads (Sigma) and incubated with bacterial protein extracts containing 20 µg His-tagged fusion protein in IP-buffer (50 mM Hepes pH 7.4, 150 mM NaCl, 1.5 mM MgC12, 1 mM EGTA, 20 mM NaF, 10% Glycerol, 1% NP-40 and protease inhibitors) at 7°C for 4 h. Then, beads were washed 4 times with IP-buffer and incubated with 100 µl 4x SDS gel loading buffer at 95°C for 5 min. Proteins were analyzed by SDS-PAGE and Western blotting using anti-GST (HRP-conjugate, Amersham Biosciences) and anti-His (Penta-His HRP-conjugate, Qiagen) antibodies.

**Mass spectrometry**. For identification, proteins of interest were separated by SDS-PAGE. Protein bands were excised from the gels and digested with trypsin. The peptide mixture was analyzed by peptide mass fingerprinting using a MALDI TofSpec2E mass spectrometer (Micromass). In case of equivocal results, the identification was performed with MS/MS sequencing. The MS/MS measurement was achieved with a nano-electrospray hybrid quadrupole mass spectrometer Q-Tof (Micromass). The Mascot software package (Matrix Science) was used for protein identification.

**Y2H analysis**. L40ccU MATa and L40ccα MATα yeast strains were transformed with plasmids encoding bait and prey proteins, respectively (24). For interaction mating, the MATα and MATa strains were individually mixed in 96-well microtiter plates, transferred onto YPD agar plates using the K2-003 spotting robot (KBiosystems) and incubated for 36 h at 30°C. After mating, the clones were transferred onto SDII (-Leu-Trp) agar plates and grown for 50 h at 30°C to select for diploid yeast strains. For selection of interactions, diploid yeasts were spotted onto SDIV (-Leu-Trp-Ura-His) agar plates as well as on nylon membranes placed on SDIV agar plates. After 7 days of incubation at 30°C, the agar plates and nylon membranes were assessed for growth and β-galactosidase activity, respectively (24).

**Co-immunoprecipitation and co-localization studies.** Co-immunoprecipitation experiments were performed as previously described (21). For immunofluorescence microscopy, COS-1 cells were grown on cover slips and co-transfected with pTL-VCP and pTL-HA-AMFR encoding full-length VCP and C-terminal HA-tagged AMFR (aa 396-643). 40 h post-transfection, cells were treated with 2% paraformaldehyde. Immunolabeling was performed with rabbit anti-VCP (1:500) coupled to CY3-conjugated antibody (red) (1:100, Dianova) and with mouse anti-HA antibody (1:200, Babco) coupled to Alexa 488-conjugated antibody (green) (1:100, Molecular Probes). Nuclei were counterstained with Hoechst (Sigma). Samples were observed with the fluorescence microscope Axioplan-2 (Zeiss).

**Ubiquitination assays.** Ubiquitination of caytaxin in vitro was performed essentially as described previously for Raf-1 (25). All reactions received 5% of protein extract from bacteria expressing His-tagged caytaxin and 1.25 mg/ml of purified ubiquitin (Sigma). When indicated 0.1 µM E1, 4 µM UbcH5b, 3 µM CHIP, 0.3 µM Hsp40 and 3 µM Hsc70 were added. Samples were incubated for 2 h at 30°C and subsequently analysed by SDS-PAGE and immunoblotting using an anti-His antibody (Hexa-His, Roche).

**Peptide arrays**. Two identical arrays were synthesized on a CAPE membrane using the Spot technology as described (26). Each array represents four scans of overlapping 15-mer peptides with a shift of three amino acids representing the interaction partners (Table 1). Probing the array towards binding to amphiphysin II was performed as described (26), except that the peroxidase-labeled anti-GST antibody (RPN1236, Amersham Biosciences) was used for detection. To exclude false positive results in the binding experiment, one array was pre-examined with GST/anti-GST antibody. All spot signal intensities (BLU) were measured on a Lumi-ImagerTM (Roche Diagnostics) and evaluated by using the software Genespotter® (Microdiscovery).

### RESULTS

### Protein arrays and overlay assays

In order to produce protein arrays for interaction studies, a human fetal brain cDNA library (hEx1) allowing the expression of His-tagged fusion proteins was used (20). cDNA inserts of 13,824 expression plasmids were sequenced at their 5'-ends, and the sequence information was stored in a database. BLASTP searches in protein databases (27) and bioinformatic analysis of sequence data revealed that about 64% of the cDNA fragments encoded human proteins in the correct reading frame, 19% of which were full-length.

For production of protein arrays, the E. coli clones were expressed in 384-well microtiter plates by addition of IPTG to the culture medium, and cells were lysed under non-denaturating conditions using lysozyme. After removal of insoluble cell debris by centrifugation, crude bacterial protein extracts were double gridded onto membrane filters using a robot. The resulting high-density spotted membranes (225 x 225 mm in size) containing 13,824 protein extracts were dried and stored at 4°C prior to protein interaction screens.

To determine how many of the protein samples on each membrane contain a recombinant His-tagged human fusion protein, the filter membranes were first probed with an anti-His antibody. Image analysis revealed that approximately 22% of the expression clones produced a detectable fusion protein. This is in agreement with previous studies demonstrating that approximately 20% of the hEx1 library clones express a soluble human protein (28). In total, about 3,000 His-tagged recombinant proteins were accessible for interaction studies on each high-density spotted filter membrane.

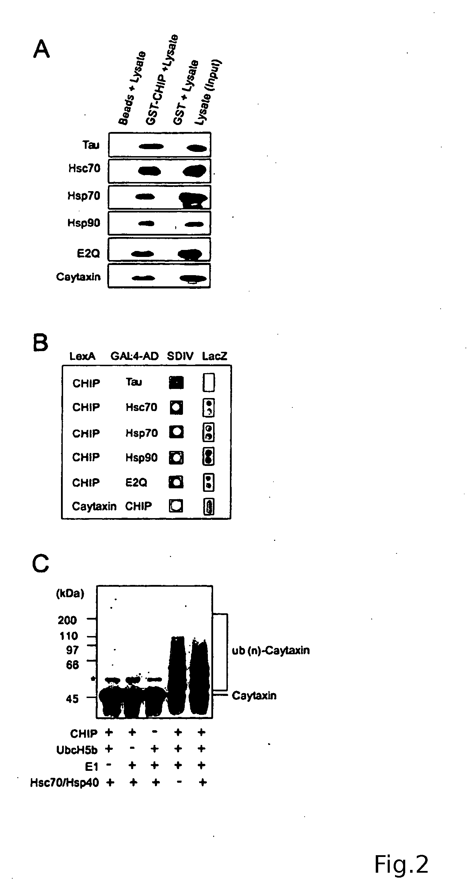

To identify PPIs, the protein arrays were used for proof-of-principle overlay experiments with the human proteins CHIP, amphiphysin II and VCP (Table 1). GST-tagged fusions were expressed in E. coli, and after native lysis with lysozyme, crude protein extracts were prepared and incubated overnight with the high-density spotted filter membranes (Fig. 1A). After extensive washing, PPIs were detected by enzyme-linked immunosorbent assay (ELISA) using an anti-GST antibody. Specific double spots that gave signals above background were detected with GST-CHIP fusion protein (Fig 1B and C), but not with the control protein GST (data not shown). As shown in Figure 1C, 27 clones containing potential CHIP-interacting proteins reached relative intensities greater than three times the standard deviation of the background, 20 of which were further characterized using independent binding assays (see below). This indicates that PPIs can be identified with this array-based overlay procedure using cell extracts containing recombinant human proteins. Similar results were obtained for the GST fusions of amphiphysin II and VCP (Supplemental Figs. 1 and 2). For each GST fusion protein three independent overlay experiments were performed, which essentially gave the same results (data not shown). The identity of the His-tagged proteins detected by overlay screening was determined using image analysis tools directly linked to our sequence database. In addition, interacting proteins expressed in E. coli were affinity purified with Ni-NTA agarose beads and analysed by mass spectrometry (MS) (Table 1).

### Identification and verification of CHIP protein-protein interactions

The co-chaperone CHIP, which is known to interact with Hsp70, Hsc70 and Hsp90 (29,30), was selected as a probe for the overlay experiments, because recent studies have demonstrated that it also functions as an E3 ubiquitin ligase and facilitates the polyubiquitination of chaperone substrates. However, the mode of action as well as the interaction partners of CHIP in this process are largely unclear.

As foreseen, the well characterized interaction partners Hsp70, Hsc70 and Hsp90 were detected by the GST-CHIP overlay assay (Table 1, Fig. 1). We identified 9 different expression clones for Hsc70 and 4 for Hsp70. 4 clones expressing fragments of Hsp90 were detected. In agreement with previous studies for all chaperones, the CHIP binding region was mapped to the C-terminus in each protein (data not shown). In addition to the known interactions, an association of CHIP with the microtubule associated protein Tau was identified by the overlay screening (Table 1). Previous studies have demonstrated that CHIP forms a protein complex with Tau in mammalian cells (29). However, a direct association has not been described. Here, we show that CHIP interacts with a domain at the C-terminus of Tau (aa 685-756), which is located downstream of the tubulin binding domains (aa 559-684). We also found that the uncharacterized ubiquitin-conjugating enzyme E2Q as well as the disease protein caytaxin (30) interact with CHIP in the overlay screen (Table 1).

To confirm the CHIP interactions, pull-down as well as yeast two-hybrid (Y2H) assays were performed (Fig. 2A and B). For pull-down experiments GST-CHIP fusion protein was expressed in E. coli and bound to agarose beads. Then, the immobilized beads were incubated with bacterial protein extracts containing the respective His-tagged interaction partners. After extensive washing, bound His-tagged proteins were detected by SDS-PAGE and immunoblotting using an anti-His antibody. As shown in Figure 2A the recombinant proteins Tau, Hsc70, Hsp70, Hsp90, E2Q and caytaxin interact with GST-CHIP in pull-down assays but not with the control protein GST or the affinity matrix.

For the two-hybrid analysis, the cDNA fragments encoding His- and GST-tagged fusions were subcloned into DNA-binding domain (DBD) and activation domain (AD) vectors and the resulting plasmids were transformed into MATa and MAT□ yeast strains for interaction mating (24). Diploid yeast clones were then selected for growth and □-galactosidase activity (24). Except the Tau/CHIP interaction, all PPIs could be confirmed by the two-hybrid assays (Fig. 2B), indicating that protein complexes not only form in vitro but also in vivo. We suggest that failure to detect the Tau/CHIP PPI could be due to insufficient translocation of the microtubule binding protein Tau into the nucleus, which is a prerequisite for reporter gene activation.

### Functional analysis of the CHIP/caytaxin interaction

Previous studies have shown that CHIP can function as an E3 ligase (25,29). Using a cell-free assay (Fig. 2C), we tested whether the protein can stimulate the ubiquitination of potential interaction partners such as caytaxin. Caytaxin was incubated for 2 h with CHIP, UbcH5b (E2) and E1 in vitro and the formation of higher molecular weight ubiquitinated caytaxin molecules was monitored by western blotting. As shown in Figure 2C, CHIP efficiently stimulated the ubiquitination of caytaxin, indicating that the protein is a substrate for the E3 ligase activity of CHIP and the interaction between both proteins could be important for regulation of caytaxin turnover in vivo. Surprisingly, caytaxin ubiquitination proceeded with similar efficiency in the absence or presence of Hsc70 (Fig. 2C). This is in marked contrast to findings obtained for other known substrates of the CHIP ubiquitin ligase, the ubiquitylation of which was highly dependent on the presence of Hsc70 (25,29). Caytaxin is the first substrate of CHIP identified to be directly recognized by the ubiquitin ligase without an involvement of Hsc70.

### Identification of amphiphysin II interaction partners and mapping of potential binding domains

Using the conserved C-terminal Src homology 3 (SH3) domain (aa 497-593) as a probe for overlay screens, we next searched for amphiphysin II interaction partners (Supplemental Fig. 1). Amphiphysin II belongs to a family of adaptor proteins that regulates synaptic vesicle endocytosis in neurons and binds to the proteins synaptojanin and dynamin (31). In addition, experimental evidence has been presented showing isoforms of amphiphysin II to associate with nuclear proteins and exhibit tumor suppressor and proapoptotic properties (32). Hence, amphiphysin II may have multiple functions in distinct subcellular processes and tissues.

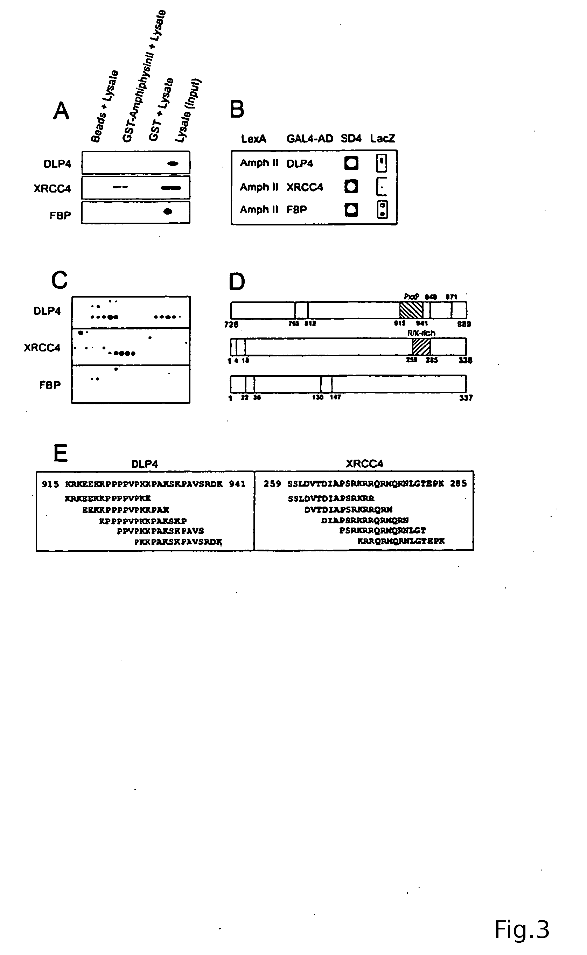

Overlay experiments with the SH3 domain of amphiphysin II allowed the identification of the known interaction partner dynamin (Table 1) (Synaptojanin expression clones were not included in the library.). In addition, the proteins DLP4 (discs large-associated protein 4), XRCC4 (DNA-repair protein XRCC4) and FBP (fructose-1,6-bisphosphatase) (Table 1) were identified as novel amphiphysin II interaction partners, and verified by in vitro pull-down and two-hybrid assays (Fig. 3A and B).

To map the binding regions in the amphiphysin II interaction partners DLP4, XRCC4 and FBP, peptide arrays on cellulose acetate membranes were used (26,33). Overlapping peptides of the proteins were incubated with the GST-amphiphysin II SH3 domain (aa 497-593) and the interacting peptide sequences were detected using an anti-GST antibody (Fig. 3C). We found that in the protein DLP4, besides a proline-rich motif (aa 915-941; Fig. 3E) with a well known PxxP core element (34), also the sequences RRDGYWFLKLLQAET (aa 798-812) and KQRQEARKRLLAAKRAASVRQNSA (aa 948-971) were recognized by the SH3 domain of amphiphysin II (Fig. 3C and D). Interestingly, in XRCC4 arginine (R)- and lysine (K)-rich sequences were found to be critical for the association with the SH3 domain (Fig. 3C-E). Besides the R/K-rich sequence in XRCC4 (aa 259-285; Fig. 3E) also the peptide KISRIHLVSEPSITH (aa 4-18) gave a signal above background (Fig. 3C and D). In FBP, the peptides RKARGTGELTQLLNS (aa 22-36) and GTIFGIYRKKSTDEP (aa 130-147) were detected by overlay screens (Fig. 3C and D). This indicates that the SH3 domain of amphiphysin II recognizes proline-rich regions with the consensus sequence PxxP for class I and II ligands (34), very basic R/K-rich regions (35) as well as peptide sequences without an obvious binding motif.

### Identification and characterization of VCP protein-protein interactions

Using the array-based overlay assay, we also screened for interaction partners of the valosin-containing protein (VCP), which is a member of the AAA ATPase family and forms highly ordered hexameric rings (36). Several lines of evidence suggest that binding of the hexamer to cofactors such as p47 (37) determines the specific role of VCP in the cell. Thus, the identification of novel VCP binding partners might help to elucidate its function in distinct subcellular processes.

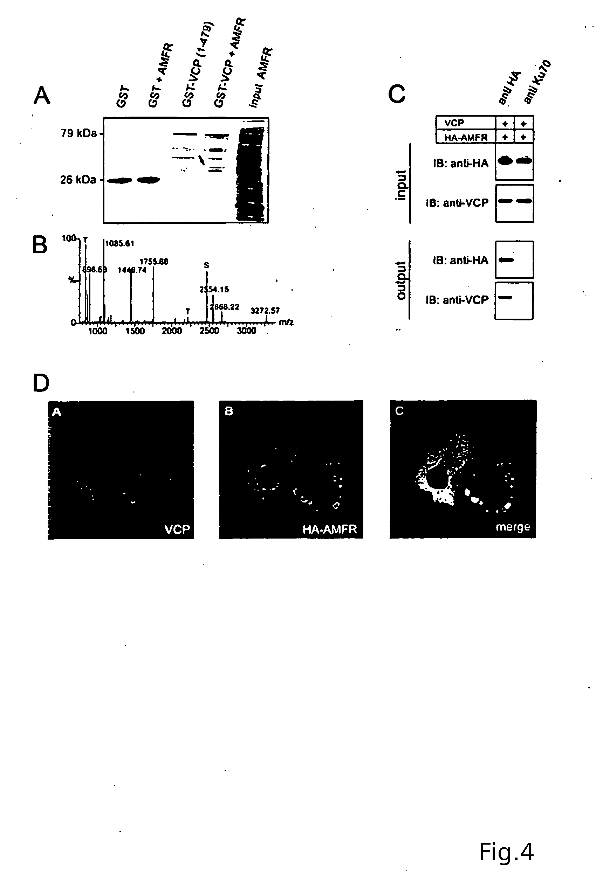

Overlay experiments with VCP (Supplemental Fig. 2) lead to the identification of the well known interaction partner p47 (38) (Table 1). Also, an association with the autocrine motility factor receptor (AMFR), only found very recently to interact with VCP (39), was detected (Table 1). We focused on the validation of the VCP/AMFR interaction in more detail using binding experiments, co-immunoprecipitations, and co-localization studies (Fig. 4). Figure 4A shows the selective enrichment on the GST-VCP affinity matrix of a 35 kDa C-terminal His-tagged AMFR fragment from a crude E. coli protein extract. In strong contrast, no AMFR protein was bound to the matrix with the control protein GST. The identity of the affinity purified AMFR protein was determined by mass spectrometry (Fig. 4B) as well as immunoblotting using specific anti-AMFR or anti-His antibodies (data not shown).

The interaction between VCP and AMFR was also detected by co-immunoprecipitation experiments using transiently transfected COS-1 cells co-expressing full-length VCP and an N-terminally truncated HA-tagged AMFR fragment. As shown in Figure 4C, a protein complex containing VCP/HA-AMFR was precipitated with the anti-HA antibody, but not with the anti-Ku70 antibody (negative control), indicating that both proteins associate with each other in mammalian cells.

The interaction between VCP and HA-AMFR was also confirmed by indirect immunofluorescence microscopy in transiently transfected COS-1 cells (Fig. 4D). Co-localization of VCP, labeled with CY3 (red) and AMFR, labeled with Alexa 488 (green) is indicated by the yellow color when the cell is observed under a triple filter. Both proteins co-localized in the cytoplasm as well as in membranous structures in the perinuclear region of COS-1 cells (Fig. 4D).

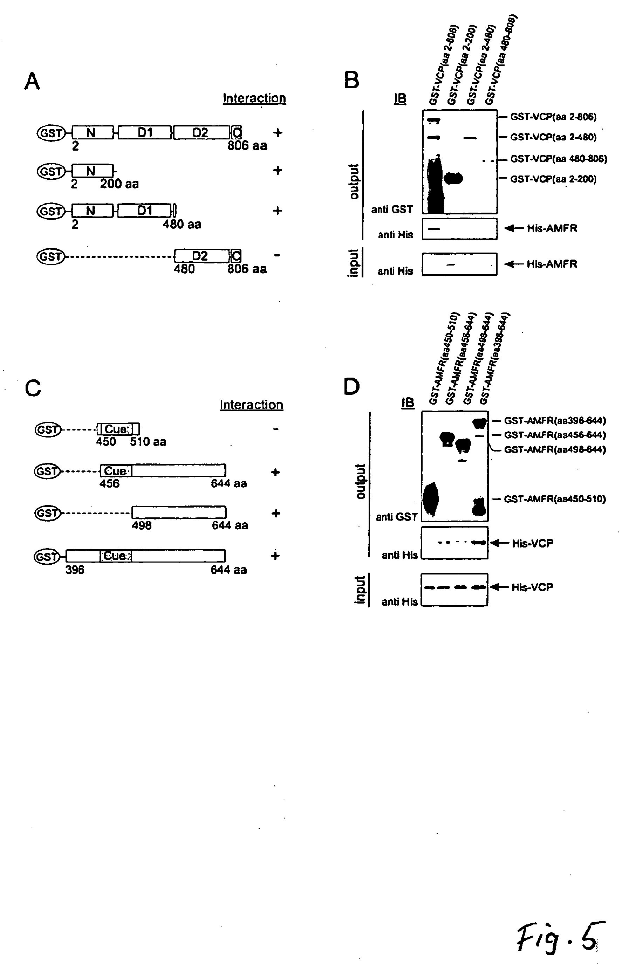

Finally, we mapped the regions critical for the protein-protein interaction in VCP and AMFR using GST pull-down assays (Fig. 5). We found that the N-terminal region of VCP (aa 1-199) is necessary and sufficient for the association with His-AMFR in vitro, while the C-terminal AAA domains D1 and D2, critical for ATP binding and hydrolysis (36), are not required for the protein-protein interaction (Fig. 5A and B). The VCP binding region in AMFR was mapped downstream of the Cue domain to the C-terminus of the protein (aa 498-643) (Fig. 5C and D).

### ABBREVIATIONS

AD, activation domain;
ATCC, American Type Culture Collection;
DBD, DNA-binding domain;
ELISA, enzyme-linked immunosorbent assay;
HA, hemagglutinin;
IPTG, isopropyl-thiogalactoside;
MS, mass spectrometry;
PPIs, protein-protein interactions;
RT, room temperature;
RZPD, Deutsches Ressourcenzentrum für Genomforschung GmbH;
SH3, Src homology 3.

### FIGURE LEGENDS

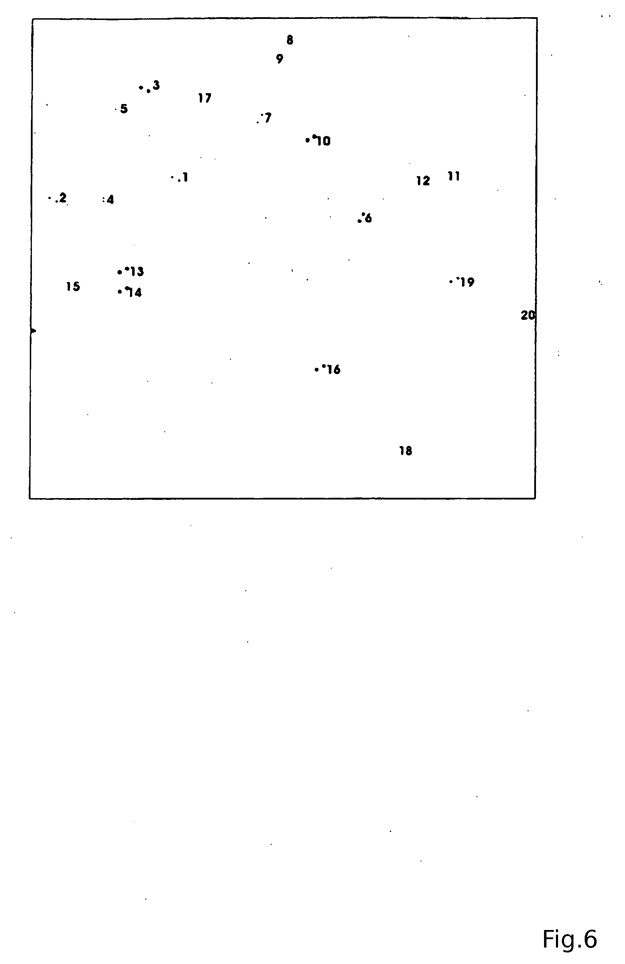

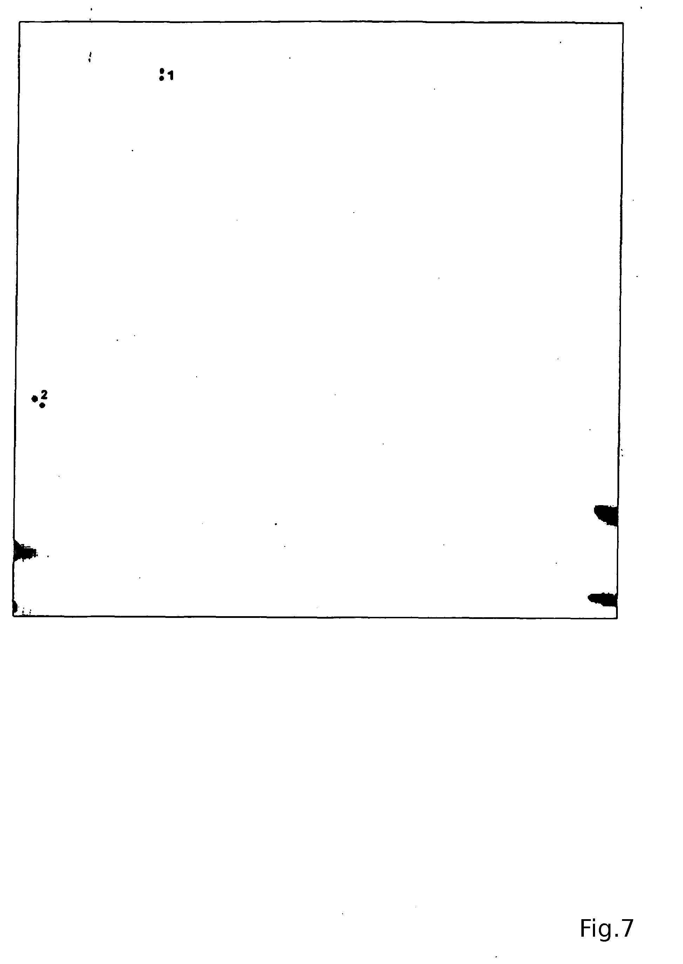

**Figure 1**. PPI screening using protein arrays. (A) Schematic overview of the screening procedure. Crude bacterial protein extracts containing His-tagged human fusion proteins are double spotted onto filter membranes using a 5x5 gridding pattern. PPIs are detected after overlay with bacterial protein extracts containing recombinant human GST fusion protein using HRP-conjugated anti-GST antibody. (B) Identification of interaction partners (double spots) of GST-CHIP by overlay screening. (C) Relative intensities of means of double spots calculated from the image shown in (B). The black horizontal line represents the triple standard deviation of the average background signals. Only clones showing relative intensities above this value in three independent experiments were used for further studies.
**Figure 2**. Verification of CHIP PPIs identified by overlay screens. (A) In vitro binding assays. GST-tagged proteins bound to glutathione-sepharose beads were incubated with bacterial protein extracts containing His-tagged human fusion proteins. After SDS-PAGE and immunoblotting, bound proteins were detected with anti-His antibody. (B) Confirmation of interactions by Y2H assays. MATa and MATα yeast strains expressing pair-wise combinations of LexA and GAL4 fusions were mated on YPD plates, and yeast strains containing interactions were selected on SDIV (-Trp-Leu-His-Ura) agar plates. In parallel, yeast colonies were spotted onto nylon membranes for detection of β-galactosidase activity (blue staining). (C) Caytaxin is a substrate of the CHIP ubiquitin ligase. Lysates from caytaxin-expressing bacteria were subjected to in vitro ubiquitination reactions in the presence of purified components of the CHIP ubiquitination machinery. Detection of caytaxin and its ubiquitinated forms was performed by immunoblotting using a hexahistidine-specific antibody. The asterisk denotes a bacterial polypeptide that crossreacts with the anti-His antibody.
**Figure 3**. Verification of amphiphysin II PPIs identified by overlay screens. (A) In vitro binding assays. (B) Yeast two-hybrid assays. (C) Screening of protein-peptide interactions. Overlapping 15-mer peptides with a shift of three amino acids representing the proteins DLP4, XRCC4 and FBP were synthesized on membranes and incubated with GST-amphiphysin II (aa 497-593) to identify interacting amino acid sequences. Protein-peptide interactions were detected with anti-GST antibody (black spots). (D) Schematic representation of the GST-amphiphysin II binding regions in the proteins DLP4, XRCC4 and FBP (boxes; aa positions are indicated by numbers). The known interaction motif PxxP (34), and R/K-rich sequences are indicated. (E) Sequences of the GST-amphiphysin II binding peptides in the indicated regions of the proteins DLP4 and XRCC4. PxxP motifs (34) in DLP4 as well as R/K-rich sequences in XRCC4 are assigned in red.
**Figure 4**. Verification of the VCP/AMFR interaction identified by overlay screens. (A) In vitro pull-down assay using GST-VCP for the detection of His-AMFR (aa 396-643) in bacterial protein extracts. The arrow indicates the His-AMFR band. (B) Identification of His-AMFR after pull-down with MALDI-TOF MS. (C) Co-immunoprecipitation of a HA-AMFR/VCP protein complex from protein extracts prepared from transiently transfected COS-1 cells using an anti-HA antibody. Anti-Ku70 antibody was used as negative control. (D) Immunofluorescence microscopy. Co-localization of VCP and HA-AMFR in transiently transfected COS-1 cells. Co-localization of the proteins is indicated by the yellow colour.
**Figure 5**. Mapping of the binding region in VCP and AMFR responsible for the PPI by in vitro pull-down experiments. (A, C) Schematic representation of the various GST-VCP and GST-AMFR fusion proteins used for in vitro binding assays and summary of the results of in vitro binding assays. Various GST-VCP (B) or GST-AMFR proteins (D) were bound to glutathione agarose beads and incubated with recombinant His-AMFR or His-VCP, respectively. Bound proteins were detected by immunoblotting using anti-GST (top panel) and anti-His antibody (middle panel). The bottom panel shows 10% of the input mixture also subjected to immunoblotting using anti-His antibody.
**Supplemental** **Figure 1** **(****Fig. 6****).** Identification of interaction partners (double spots) of GST-SH3 domain of amphiphysin II by overlay screening.
**Supplemental** **Figure 2** **(****Fig. 7****).** Identification of interaction partners (double spots) of GST-VCP by overlay screening.

### REFERENCES

1. Walhout, A. J., and Vidal, M. (2001) Protein interaction maps for model organisms. Nat. Rev. Mol. Cell Biol. 2, 55-62
2. Legrain, P., Wojcik, J., and Gauthier, J. M. (2001) Protein--protein interaction maps: a lead towards cellular functions. Trends Genet. 17, 346-52
3. Drees, B. L., Sundin, B., Brazeau, E., Caviston, J. P., Chen, G. C., Guo, W., Kozminski, K. G., Lau, M. W., Moskow, J. J., Tong, A., Schenkman, L. R., McKenzie, A., 3rd, Brennwald, P., Longtine, M., Bi, E., Chan, C., Novick, P., Boone, C., Pringle, J. R., Davis, T. N., Fields, S., and Drubin, D. G. (2001) A protein interaction map for cell polarity development. J. Cell Biol. 154, 549-71
4. Parsons, M., Vojnovic, B., and Ameer-Beg, S. (2004) Imaging protein-protein interactions in cell motility using fluorescence resonance energy transfer (FRET). Biochem. Soc. Trans. 32, 431-3
5. Emili, A. Q., and Cagney, G. (2000) Large-scale functional analysis using peptide or protein arrays. Nat. Biotechnol. 18, 393-7
6. Grayhack, E. J., and Phizicky, E. M. (2001) Genomic analysis of biochemical function. Curr. Opin. Chem. Biol. 5, 34-9
7. Zhu, H., and Snyder, M. (2003) Protein chip technology. Curr. Opin. Chem. Biol. 7, 55-63
8. Feilner, T., Kreutzberger, J., Niemann, B., Kramer, A., Possling, A., Seitz, H., and Kersten, B. (2004) Proteomic studies using microarrays. Curr. Proteomics 1, 283-295
9. Labaer, J., and Ramachandran, N. (2005) Protein microarrays as tools for functional proteomics. Curr. Opin. Chem. Biol. 9, 14-9
10. Poetz, O., Schwenk, J. M., Kramer, S., Stoll, D., Templin, M. F., and Joos, T. O. (2005) Protein microarrays: catching the proteome. Mech. Ageing Dev. 126, 161-70
11. Angenendt, P. (2005) Progress in Protein and Antibody Microarray Technology. Drug Discov. Today: Targets 10, 503-511
12. Ramachandran, N., Hainsworth, E., Bhullar, B., Eisenstein, S., Rosen, B., Lau, A. Y., Walter, J. C., and LaBaer, J. (2004) Self-assembling protein microarrays. Science 305, 86-90
13. Zhu, H., Bilgin, M., Bangham, R., Hall, D., Casamayor, A., Bertone, P., Lan, N., Jansen, R., Bidlingmaier, S., Houfek, T., Mitchell, T., Miller, P., Dean, R. A., Gerstein, M., and Snyder, M. (2001) Global analysis of protein activities using proteome chips. Science 293, 2101-5
14. Hall, D. A., Zhu, H., Zhu, X., Royce, T., Gerstein, M., and Snyder, M. (2004) Regulation of gene expression by a metabolic enzyme. Science 306, 482-4
15. Kersten, B., Possling, A., Blaesing, F., Mirgorodskaya, E., Gobom, J., and Seitz, H. (2004) Protein microarray technology and ultraviolet crosslinking combined with mass spectrometry for the analysis of protein-DNA interactions. Anal. Biochem. 331, 303-313
16. Michaud, G. A., Salcius, M., Zhou, F., Bangham, R., Bonin, J., Guo, H., Snyder, M., Predki, P. F., and Schweitzer, B. I. (2003) Analyzing antibody specificity with whole proteome microarrays. Nat. Biotechnol. 21, 1509-12
17. Martzen, M. R., McCraith, S. M., Spinelli, S. L., Torres, F. M., Fields, S., Grayhack, E. J., and Phizicky, E. M. (1999) A biochemical genomics approach for identifying genes by the activity of their products. Science 286, 1153-5
18. Zhu, H., Klemic, J. F., Chang, S., Bertone, P., Casamayor, A., Klemic, K. G., Smith, D., Gerstein, M., Reed, M. A., and Snyder, M. (2000) Analysis of yeast protein kinases using protein chips. Nat. Genet. 26, 283-9
19. Kramer, A., Feilner, T., Possling, A., Radchuk, V., Weschke, W., Burkle, L., and Kersten, B. (2004) Identification of barley CK2alpha targets by using the protein microarray technology. Phytochem. 65, 1777-84
20. Büssow, K., Cahill, D., Nietfeld, W., Bancroft, D., Scherzinger, E., Lehrach, H., and Walter, G. (1998) A method for global protein expression and antibody screening on high-density filters of an arrayed cDNA library. Nucleic Acids Res. 26, 5007-8
21. Sittler, A., Walter, S., Wedemeyer, N., Hasenbank, R., Scherzinger, E., Eickhoff, H., Bates, G. P., Lehrach, H., and Wanker, E. E. (1998) SH3GL3 associates with the Huntingtin exon 1 protein and promotes the formation of polygln-containing protein aggregates. Mol. Cell 2, 427-36
22. Wanker, E. E., Rovira, C., Scherzinger, E., Hasenbank, R., Walter, S., Tait, D., Colicelli, J., and Lehrach, H. (1997) HIP-I: a huntingtin interacting protein isolated by the yeast two-hybrid system. Hum. Mol. Genet. 6, 487-95
23. Wanker, E. E., Scherzinger, E., Heiser, V., Sittler, A., Eickhoff, H., and Lehrach, H. (1999) Membrane filter assay for detection of amyloid-like polyglutamine-containing protein aggregates. Methods Enzymol. 309, 375-86
24. Goehler, H., Lalowski, M., Stelzl, U., Waelter, S., Stroedicke, M., Worm, U., Droege, A., Lindenberg, K. S., Knoblich, M., Haenig, C., Herbst, M., Suopanki, J., Scherzinger, E., Abraham, C., Bauer, B., Hasenbank, R., Fritzsche, A., Ludewig, A. H., Buessow, K., Coleman, S. H., Gutekunst, C. A., Landwehrmeyer, B. G., Lehrach, H., and Wanker, E. E. (2004) A Protein Interaction Network Links GIT1, an Enhancer of Huntingtin Aggregation, to Huntington's Disease. Mol. Cell 15, 853-65
25. Demand, J., Alberti, S., Patterson, C., and Hohfeld, J. (2001) Cooperation of a ubiquitin domain protein and an E3 ubiquitin ligase during chaperone/proteasome coupling. Curr. Biol. 11, 1569-77
26. Landgraf, C., Panni, S., Montecchi-Palazzi, L., Castagnoli, L., Schneider-Mergeiier, J., Volkmer-Engert, R., and Cesareni, G. (2004) Protein interaction networks by proteome Peptide scanning. PLoS Biol. 2, E14
27. Altschul, S. F., Gish, W., Miller, W., Myers, E. W., and Lipman, D. J. (1990) Basic local alignment search tool. J. Mol. Biol. 215, 403-10
28. Bussow, K., Quedenau, C., Sievert, V., Tischer, J., Scheich, C., Seitz, H., Hieke, B., Niesen, F. H., Gotz, F., Harttig, U., and Lehrach, H. (2004) A catalog of human cDNA expression clones and its application to structural genomics. Genome Biol. 5, R71
29. Petrucelli, L., Dickson, D., Kehoe, K., Taylor, J., Snyder, H., Grover, A., De Lucia, M., McGowan, E., Lewis, J., Prihar, G., Kim, J., Dillmann, W. H., Browne, S. E., Hall, A., Voellmy, R., Tsuboi, Y., Dawson, T. M., Wolozin, B., Hardy, J., and Hutton, M. (2004) CHIP and Hsp70 regulate tau ubiquitination, degradation and aggregation. Hum. Mol. Genet. 13, 703-14
30. Bomar, J. M., Benke, P. J., Slattery, E. L., Puttagunta, R., Taylor, L. P., Seong, E., Nystuen, A., Chen, W., Albin, R. L., Patel, P. D., Kittles, R. A., Sheffield, V. C., and Burmeister, M. (2003) Mutations in a novel gene encoding a CRAL-TRIO domain cause human Cayman ataxia and ataxia/dystonia in the jittery mouse. Nat. Genet. 35, 264-9
31. Wigge, P., and McMahon, H. T. (1998) The amphiphysin family of proteins and their role in endocytosis at the synapse. Trends Neurosci. 21, 339-44
32. DuHadaway, J. B., Sakamuro, D., Ewert, D. L., and Prendergast, G. C. (2001) Binl mediates apoptosis by c-Myc in transformed primary cells. Cancer Res. 61, 3151-6
33. Reineke, U., Volkmer-Engert, R., and Schneider-Mergerier, J. (2001) Applications of peptide arrays prepared by the SPOT-technology. Curr. Opin. Biotechnol. 12, 59-64
34. Tong, A. H., Drees, B., Nardelli, G., Bader, G. D., Brannetti, B., Castagnoli, L., Evangelista, M., Ferracuti, S., Nelson, B., Paoluzi, S., Quondam, M., Zucconi, A., Hogue, C. W., Fields, S., Boone, C., and Cesareni, G. (2002) A combined experimental and computational strategy to define protein interaction networks for peptide recognition modules. Science 295, 321-4
35. Liu, Q., Berry, D., Nash, P., Pawson, T., McGlade, C. J., and Li, S. S. (2003) Structural basis for specific binding of the Gads SH3 domain to an RxxK motif-containing SLP-76 peptide: a novel mode of peptide recognition. Mol. Cell 11, 471-81
36. Rouiller, I., DeLaBarre, B., May, A. P., Weis, W. I., Brunger, A. T., Milligan, R. A., and Wilson-Kubalek, E. M. (2002) Conformational changes of the multifunction p97 AAA ATPase during its ATPase cycle. Nat. Struct. Biol. 9, 950-7
37. Kondo, H., Rabouille, C., Newman, R., Levine, T. P., Pappin, D., Freemont, P., and Warren, G. (1997) p47 is a cofactor for p97-mediated membrane fusion. Nature 388, 75-8
38. Bruderer, R. M., Brasseur, C., and Meyer, H. H. (2004) The AAA ATPase p97/VCP interacts with its alternative co-factors, Ufdl-Npl4 and p47, through a common bipartite binding mechanism. J. Biol. Chem. 279, 49609-16
39. Zhong, X., Shen, Y., Ballar, P., Apostolou, A., Agami, R., and Fang, S. (2004) AAA ATPase p97/VCP interacts with gp78: a ubiquitin ligase for ER-associated degradation. J. Biol. Chem. 279, 45676-84
40. Ballinger, C. A., Connell, P., Wu, Y., Hu, Z., Thompson, L. J., Yin, L. Y., and Patterson, C. (1999) Identification of CHIP, a novel tetratricopeptide repeat-containing protein that interacts with heat shock proteins and negatively regulates chaperone functions. Mol. Cell Biol. 19, 4535-45
41. Cyr, D. M., Hohfeld, J., and Patterson, C. (2002) Protein quality control: U-box-containing E3 ubiquitin ligases join the fold. Trends Biochem. Sci. 27, 368-75
42. Zelhof, A. C., Bao, H., Hardy, R. W., Razzaq, A., Zhang, B., and Doe, C. Q. (2001) Drosophila Amphiphysin is implicated in protein localization and membrane morphogenesis but not in synaptic vesicle endocytosis. Development 128, 5005-15
43. Berg, J. M., Tymoczko, J. L., and Stryer, L. (2003), Biochemie, 5th Ed., pp. 1-1200, Spektrum Akademischer Verlag, Heidelberg
44. Boggon, T. J., and Eck, M. J. (2004) Structure and regulation of Src family kinases. Oncogene 23, 7918-27
45. Rabinovich, E., Kerem, A., Frohlich, K. U., Diamant, N., and Bar-Nun, S. (2002) AAA-ATPase p97/Cdc48p, a cytosolic chaperone required for endoplasmic reticulum-associated protein degradation. Mol. Cell Biol. 22, 626-34
46. Meyer, H. H., Wang, Y., and Warren, G. (2002) Direct binding of ubiquitin conjugates by the mammalian p97 adaptor complexes, p47 and Ufd1-Npl4. Embo J. 21, 5645-52
47. Lueking, A, Konthur, Z., Eickhoff, H., Büssow, K., Lehrach, H. and Cahill, D.J. (2001) Protein Microarrays - A Tool fort he Post-Genomic Era. Curr. Genomics 2, 151-159.

**Table 1. Protein interaction partners of CHIP, amphiphysin II and VCP identified by overlay experiments with protein arrays.**

| **Gene name** | **Gene ID** | **Protein name** | **Acc. Number** | **Over lay** | **Pull-down** | **Y2H** | **Sequence (aa)** | **MS** |
|---|---|---|---|---|---|---|---|---|
| *STUB1* | 10273 | Carboy terminus of Hsp70-interacting protein (CHIP) | Q9UNE7 | | | | 2 - 303 | 5 |
| *MAPT* | 4137 | Microtubule-associated protein tau (Tau) | P1063 | + | + | - | 685 - 756 | 2 |
| *HSPA8* | 3312 | Heat shock cognate 71 kDa protein (Hsc 70) | P11142 | + | + | + | 417 - 646 | 12 |
| *HSPAIB* | 3304 | Heat shock 70 kDa protein 1 (Hsp 70) | P08107 | + | + | + | 70 - 641 | 21 |
| *HSP90A* | 30591 | Heat shock protein HSP90-alpha (Hsp 90) | P07900 | + | + | + | 227 - 378 | 7 |
| *UBE2Q* | 55585 | Ubiquitin-conjugating enzyme E2Q (E2Q) | O7Z7E8 | + | + | + | 107 - 422 | 10 |
| *ATCAY* | 85300 | Caytaxin | Q86WG3 | + | + | + | 78 - 370 | 7 |
| *BINJ* | 274 | Myc box dependent interacting protein 1 (Amphiphysin II) | Q00499 | | | | 497 - 593 | 4 |
| *DNMI* | 1759 | Dynamin 1 (DNM1) | Q05193 | + | nd | nd | 634 - 864 | 2 |
| *DLGAP4* | 22839 | Discs large-associated protein 4 (DLP4) | Q9Y2H0 | + | + | + | 726 - 989 | 16 |
| *XRCC4* | 7518 | DNA-repair protein XRCC4 (XRCC4) | Q13426 | + | + | + | 1 - 336 | 7 |
| *FBPI* | 2203 | Fructose-1,6-bisphosphatase (FBP) | P09467 | + | + | + | 1 - 337 | 12 |
| *VCP* | 7415 | Valosin-containing protein (VCP) | P55072 | | | | 1-479 | 15 |
| *NSFLIC* | 55968 | NSFLI cofactor p47 (p47) | O9UNZ2 | + | + | + | 1 - 370 | 8 |
| *AMFR* | 267 | Autocrine motility factor receptor, isoform 2 (AMFR) | Q9UKV5 | + | + | + | 396 - 643 | 8 |

| | | | | | | | | |
|---|---|---|---|---|---|---|---|---|
| SwissProt accession numbers (Acc. Number) obtained by MS protein identification; +, positive result in the respective assay; -, negative result; nd, not determined. Sequence start derived from the translated cDNA insert up to the end of the protein; MS, number of peptides used for identification by MALDI peptide mass fingerprinting or ESI-MS/MS sequencing. | | | | | | | | |

## Claims

1. Method of screening of protein-protein interactions comprising the following steps
(a) providing a library of cDNA clones using a first acceptable carrier, allowing the expression of a first protein,
(b) expression of the first protein,
(c) providing a library of cDNA clones using a second acceptable carrier, allowing the expression of a second protein or
(c') providing a raw or unpurified biological probe comprising the second protein,
(d) expression of the second protein of (c),
(e) lysis of the clones used in step (a) and centrifugation/ filtration of the lysed clones, whereby a crude cell extract comprising the first protein is obtained,
(f) lysis of the clones used in step (c) and centrifugation/ filtration of the lysed clones, whereby a crude cell extract comprising the second protein is obtained,
(g) immobilisation of the crude cell extract comprising the expressed first protein obtained in step (e) using a third acceptable carrier,
(h) contacting the third carrier with the crude cell extract obtained in step (f) or with the raw as unpurified probe in step (c') comprising the second protein,
(i) detection of the interaction of the first and the second protein, whereat the interaction of proteins in step (i) is detected using an antibody against a protein-tag of the second protein obtained in step (f) and / or using an antibody against the second protein.

2. Method according to claim 1, **characterized in that,**
the clones in step (a) and / or (c) are E.coli clones, yeast clones, clones using mammalian cells or other diploid cells.

3. Method according to claim 1, **characterized in that**,
the first carrier in step (a) is a microtiter plate and/or the second carrier of step (c) is a microtiter plate or a flask and/ or the third carrier of step (g) is a membrane filter or a coated glass slide.

4. Method according to claim 1, **characterized in that**, the membrane filter used in step (g) is a nitrocellulose or a polyvinylidene difluride (PVDF) membrane.

5. Method according to claim 1, **characterized in that**,
the lysis in step (e) and/or (f) is a native cell lysis, in particular using lysozyme or sonification and nondenaturating detergents.

6. Method according to claim 1, **characterized in that,**
the crude cell extract comprising the first protein of step (e) is spotted onto a membrane or a coated glass slide.

7. Method according to claim 1, **characterized in that**,
the cell extracts in steps (e) and/or (f) are native cell extracts.

8. Method according to claim 1, **characterized in that**,
the contacting step (h) is an overlay assay.

9. Method according to claim 1, **characterized in that**, the protein-tag of the second protein obtained in step (f) is a GST-, HIS-, myc-, FLAG-, HA- or a fluorescent-tag.

10. Method according to claim 1, **characterized in that**,
the interaction of the proteins in step (i) is detected by a fluorescence or an enzyme-linked detection method or a method based on magnetic properties.

11. Method according to claim 1, **characterized in that**, the interaction of the proteins in step (i) is detected by an ELISA assay.

## Patentansprüche

1. Verfahren zum Screening von Protein-Protein-Wechselwirkungen, umfassend die folgenden Schritte:
(a) Bereitstellen einer cDNA-Klonbibliothek unter Verwendung eines ersten annehmbaren Trägers, so dass die Expression eines ersten Proteins möglich ist,
(b) Exprimieren des ersten Proteins,
(c) Bereitstellen einer cDNA-Klonbibliothek unter Verwendung eines zweiten annehmbaren Trägers, so dass die Expression eines zweiten Proteins möglich ist,
(c') Bereitstellen einer rohen oder ungereinigten biologischen Sonde, die das zweite Protein umfasst,
(d) Exprimieren des zweiten Proteins (c),
(e) Lysieren der in Schritt (a) verwendeten Klone und Zentrifugieren/Filtrieren der lysierten Klone, wodurch ein roher Zellextrakt erhalten wird, der das erste Protein umfasst,
(f) Lysieren der in Schritt (c) verwendeten Klone und Zentrifugieren/Filtrieren der lysierten Klone, wodurch ein roher Zellextrakt erhalten wird, der das zweite Protein umfasst,
(g) Immobilisieren des rohen Zellextrakts, der das in Schritt (e) erhaltene exprimierte erste Protein umfasst, wobei ein dritter annehmbarer Träger verwendet wird,
(h) Inkontaktbringen des dritten Trägers mit dem in Schritt (f) erhaltenen rohen Zellextrakt oder mit der rohen oder ungereinigten Sonde in Schritt (c'), die das zweite Protein umfassen,
(i) Nachweisen der Wechselwirkung des ersten und zweiten Proteins, wobei die Wechselwirkung der Proteine in Schritt (i) mit einem Antikörper gegen eine Protein-Markierung des in Schritt (f) erhaltenen zweiten Proteins und/oder mit einem Antikörper gegen das zweite Protein nachgewiesen wird.

2. Verfahren nach Anspruch 1,
**dadurch gekennzeichnet, dass**
die Klone in Schritt (a) und/oder (c) *E*. *coli*-Klone, Hefe-Klone, Klone unter Verwendung von Säugerzellen oder anderen diploiden Zellen sind.

3. Verfahren nach Anspruch 1,
**dadurch gekennzeichnet, dass**
der erste Träger in Schritt (a) eine Mikrotiterplatte ist und/oder der zweite Träger von Schritt (c) eine Mikrotiterplatte oder ein Kolben ist und/oder der dritte Träger von Schritt (g) ein Membranfilter oder ein beschichteter Objektträger ist.

4. Verfahren nach Anspruch 1,
**dadurch gekennzeichnet, dass**
das in Schritt (g) verwendete Membranfilter eine Nitrocellulose- oder Polyvinylidendifluorid(PVDF)-Membran ist.

5. Verfahren nach Anspruch 1,
**dadurch gekennzeichnet, dass**
die Lyse in Schritt (e) und/oder (f) eine Lyse nativer Zellen ist, insbesondere mit Lysozym oder Beschallung und nicht denaturierenden Detergenzien.

6. Verfahren nach Anspruch 1,
**dadurch gekennzeichnet, dass**
der rohe Zellextrakt, umfassend das erste Protein von Schritt (e), auf eine Membran oder einen beschichteten Objektträger aufgetüpfelt wird.

7. Verfahren nach Anspruch 1,
**dadurch gekennzeichnet, dass**
die Zellextrakte bei den Schritten (e) und/oder (f) Extrakte nativer Zellen sind.

8. Verfahren nach Anspruch 1,
**dadurch gekennzeichnet, dass**
der Schritt des Inkontaktbringens (h) ein Overlay-Assay ist.

9. Verfahren nach Anspruch 1,
**dadurch gekennzeichnet, dass**
die Protein-Markierung des in Schritt (f) erhaltenen zweiten Proteins eine GST-, His-, myc-, FLAG-, HA- oder Fluoreszenz-Markierung ist.

10. Verfahren nach Anspruch 1,
**dadurch gekennzeichnet, dass**
die Wechselwirkung der Proteine in Schritt (i) mit Hilfe eines Fluoreszenznachweisverfahrens oder eines enzymvermittelten Nachweisverfahrens oder eines Verfahrens auf der Grundlage magnetischer Eigenschaften nachgewiesen wird.

11. Verfahren nach Anspruch 1,
**dadurch gekennzeichnet, dass**
die Wechselwirkung der Proteine in Schritt (i) mit Hilfe eines ELISA nachgewiesen wird.

## Revendications

1. Méthode de détection d'interactions protéine-protéine comprenant les étapes suivantes :
(a) pourvoir à une banque de clones d'ADNc en utilisant un premier support acceptable, permettant l'expression d'une première protéine,
(b) expression de la première protéine,
(c) pourvoir à une banque de clones d'ADNc en utilisant un second support acceptable, permettant l'expression d'une seconde protéine ou
(c') pourvoir à une sonde biologique brute ou non purifiée comprenant la seconde protéine,
(d) expression de la seconde protéine de (c),
(e) lyse des clones utilisés dans l'étape (a) et centrifugation/filtration des clones lysés, au moyen de quoi on obtient un extrait cellulaire brut comprenant la première protéine,
(f) lyse des clones utilisés dans l'étape (c) et centrifugation/filtration des clones lysés, au moyen de quoi on obtient un extrait cellulaire brut comprenant la seconde protéine,
(g) fixation de l'extrait cellulaire brut comprenant la première protéine exprimée obtenue à l'étape (e) en utilisant un troisième support acceptable,
(h) mise en contact du troisième support avec l'extrait cellulaire brut obtenu à l'étape (f) ou avec la sonde brute ou non purifiée prévue à l'étape (c') comprenant la seconde protéine, et
(i) détection de l'interaction de la première et la seconde protéine, l'interaction des protéines dans l'étape (i) étant détectée en utilisant un anticorps contre un marqueur de protéine de la seconde protéine obtenue à l'étape (f) et/ou en utilisant un anticorps contre la seconde protéine.

2. Méthode selon la revendication 1, **caractérisée en ce que** les clones à l'étape (a) ou (c) sont des clones d'E. coli, des clones de levure, des clones utilisant des cellules de mammifère ou d'autres cellules diploïdes.

3. Méthode selon la revendication 1, **caractérisée en ce que** le premier support dans l'étape (a) est une plaque de micro-titration et/ou le second support dans l'étape (c) est une plaque de micro-titration ou un flacon et/ou le troisième support de l'étape (g) est un filtre à membrane ou une lame porte-objet de verre enduit.

4. Méthode selon la revendication 1, **caractérisée en ce que** le filtre à membrane utilisé dans l'étape (g) est une membrane de nitrocellulose ou de difluorure de poyvinylidène (PVDF).

5. Méthode selon la revendication 1, **caractérisée en ce que** la lyse à l'étape (e) et/ou (f) est une lyse de cellules natives, réalisée notamment au moyen de lysozyme ou par sonication et avec des détergents non dénaturants.

6. Méthode selon la revendication 1, **caractérisée en ce que** l'extrait cellulaire brut comprenant la première protéine de l'étape (e) est déposé en taches sur une membrane ou une lame porte-objet de verre enduit.

7. Méthode selon la revendication 1, **caractérisée en ce que** les extraits cellulaires dans les étapes (e) et/ou (f) sont des extraits de cellules natives.

8. Méthode selon la revendication 1, **caractérisée en ce que** l'étape de mise en contact (h) est un dosage sur couche superposée.

9. Méthode selon la revendication 1, **caractérisée en ce que** le marqueur de protéine de la seconde protéine obtenue à l'étape (f) est un marqueur GST, HIS, myc, FLAG, HA ou un marqueur fluorescent.

10. Méthode selon la revendication 1, **caractérisée en ce que** l'interaction des protéines à l'étape (i) est détectée par une méthode de détection par fluorescence ou à enzyme lié ou par une méthode basée sur les propriétés magnétiques.

11. Méthode selon la revendication 1, **caractérisée en ce que** l'interaction des protéines dans l'étape (i) est détectée par un dosage ELISA.
